# EUROPEAN PATENT SPECIFICATION

(11) **EP 0 358 657 B1**
(45) Date of publication and mention of the grant of the patent: **09.06.1993**
(21) Application number: 88903410.4
(22) Date of filing: 25.03.1988
(51) Int. Cl.: C07H 21/04, A61K 31/70

(54) **POLY(DEOXYRIBONUCLEOTIDES), PHARMACEUTICAL COMPOSITIONS, USE AND PREPARATION OF THE POLY(DEOXYRIBONUCLEOTIDES)**
POLYDEOXYRIBONUKLEOTIDE, PHARMAZEUTISCHE ZUSAMMENSETZUNGEN, VERWENDUNG UND HERSTELLUNG VON POLYDEOXYRIBONUKLEOTIDEN
POLY(DEOXYRIBONUCLEOTIDES), COMPOSITIONS PHARMACEUTIQUES, UTILISATION ET PREPARATION DES POLY(DEOXYRIBONUCLEOTIDES)

(30) Priority: 27.03.1987 NL 8700724
(43) Date of publication of application: 21.03.1990
(73) Proprietor: Buck, H.M. Prof Dr, NL-5042 PE TILBURG (NL); Koole, Ir L.H. Dr, NL-6200 MD MAASTRICHT (NL)
(72) Inventor: BUCK, Hendricus, Maria, NL-5042 PE Tilburg (NL)
(74) Representative: Smulders, Theodorus A.H.J., Ir.
(86) International application number: NL8800013
(87) International publication number: WO8807542

(56) References cited:
- EP-A- 0 090 789
- EP-A- 0 108 387
- Chemical Abstracts, vol. 105, 1986, (Columbus, Ohio, US), L H Koole et al.: "A stable parallel duplex structure for the hexamer d(TpTpTpTpTpTp) with phosphate triester linkages", see page 270, abstract no. 37632v, & Proc. K. Ned. Akad. Wet., Ser. B: Palaeontol., Geol., Phys., Chem., Anthropol. 1986, 89(1), 51-5 cited in the application
- Journal of the American Chemical Society, vol. 93, no. 24, 1 December 1971 (Columbus, Ohio, US), P S Miller et al.: "Syntheses and properties of adenine and thymine nucleoside alkyl phosphotriesters, the neutral analogs of dinucleoside mono-phosphates", pages 6657-6665, see pages 6659, 6662-3 cited in the application
- Chemical Abstracts, vol. 101, 1984, (Columbus, Ohio, US), P S Miller et al.: "Nonionic oligo-nucleotide analogs as new tools for studies on the structure and function of nucleic acids inside living cells", see page 219, abstract no. 2478q, & Jerusalem Symp. Quantum Chem. Biochem. 1983, 16 (Nucleic Acids), 521-35

## Description

This invention relates to poly(deoxyribonucleotides) comprising 8 or more deoxyribonucleoside units which are linked together by phosphate groups.

All living organisms contain deoxyribonucleic acid (DNA) in the form of large molecules built up from units designated as deoxyribonucleosides. Each deoxyribonucleoside unit consists of a sugar molecule, namely β-D-2-deoxyribofuranose, to the C¹ atom of which a base from the group consisting of adenine, guanine, thymine and cytosine is bound via the N⁹ atom of adenine or guanine or the N¹ atom of thymine or cytosine. In the polymeric DNA the deoxyribonucleoside units are linked together via phosphate groups between the 3' OH group of one nucleoside and the 5' OH group of the other nucleoside. The subunits of the polymeric DNA, said subunits consisting of a phosphate group, sugar molecule and base, are called deoxyribonucleotides, or briefly nucleotides. The polymeric DNA serves in all living creatures as a carrier of genetic information which is determined by the order (or sequence) of the bases in the molecule.

Since the structural analyses by Watson and Crick in the early fifties it has been clear that DNA occurs naturally in the form of a double helix (or duplex) consisting of two strands, which is held together by hydrogen bonds between complementary bases, i.e. between A and T and between G and C. In the meantime, different duplex forms of DNA have become known, among which the B form found by Watson and Crick is best known. Characteristic is, among other things, the fact that the two complementary strands run in opposite direction, i.e. are antiparallel. The stability of the duplex is determined by different factors, such as the length of the duplex, the ratio between the number of C-G pairs (each pair having 3 hydrogen bonds) and A-T pairs (each pair having only 2 hydrogen bonds), the sequence of the bases and the electrostatic repulsion of the phosphate groups having a negative charge under physiological conditions. Electrostatic interaction between the phosphate groups and inorganic cations, such as Mg²⁺, or basic proteins, such as histones, leads to a higher stability of the duplex. A measure of the stability of the duplex is the melting temperature Tₘ which can be determined by plotting the absorption at 260 nm against the temperature. A high Tₘ value indicates a high stability of the duplex.

In the meantime different methods have been developed with which DNA molecules can be synthesized. An elegant, automatically performable method makes use of a carrier of controlled pore glass (CPG) to which a first nucleoside is covalently bound via a spacer. This nucleoside is linked via the 3' OH group and has a 5' OH group protected by the acid-labile di-p-methoxytrityl (DMTr) group. To this is added, in as many cycles, a subunit of the DNA to be prepared. Each cycle starts with a removal of the 5' OH protecting DMTr group and reaction with a compound activated by treatment with tetrazole and having formula 3 of the sheet of formulae, in which NR₂ is a diisopropylamino group, D is the 5' OH protecting DMTr group, and X* is thymine, benzoyl adenine, benzoyl cytosine or isopropylcarbonyl guanine. After unreacted substrate has been blocked by acetylation, the methylphosphite group is oxidized with iodine to form a methylphosphate group prior to carrying out the next cycle. After all the desired cycles have taken place, the phosphate protecting methyl groups are removed by treatment with thiophenol, the bond to the CPG carrier is broken by treatment with ammonia, and the base protecting groups are removed. For clarity's sake, it is observed that the above described method is only one of the many possible synthesis variants. There are also synthesis variants with other protecting groups and variants in which the components contain no phosphite but a phosphate group.

Several attempts have already been made to synthesize poly(deoxyribonucleotides) without a negatively charged phosphate group. Miller et al, J.A.C.S. 93 (1971) 6657-6665 and Pless and Ts'O, Biochem. 16 (1977) 1239-1250 describe experiments with alkylphosphotriesters, namely the preparation of d([Tp(OEt)]₇T) containing ethyl phosphate groups between the nucleoside units. As a result of the chiral phosphorus atoms the reaction product consists of a mixture of 128 diastereomers which during hybridisation with poly(deoxyadenylate) form duplexes having divergent stabilities. It is observed that no corresponding methylphosphotriesters are described which could in general not be prepared owing to the absence of a feasible synthesis method.

The same researchers then proceeded to a synthesis of poly(deoxyribonucleotides) in which methylphosphonate groups having the formula -O-P(O)(CH₃)-O- act as linking elements between the nucleoside units: see Miller et al, Biochimie 67 (1985) 769-776; Miller et al, Jerus. Symp. Quantum Chem. Biochem. 18 (1985) 207-219; Miller et al, in "Nucleic Acids: The Vectors of Life", eds. Pullman and Jortner, Reidel Publishing Company, (1983), 521-535; Ts'O et al, in "Development of Target-Oriented Anticancer Drugs", eds. Cheng et al, Raven Press, New York (1983), 189-206; Agris et al, Biochem. 25 (1986) 6268-6275.

Hybridisation experiments of such methylphosphonate oligonucleotides with complementary normal oligonucleotides have shown that duplexes are formed, the stability of which is greater in the case of tetranucleotides than in the case of dinucleotides. The duplex stability, however, decreases with longer molecules, and with oligonucleotides haying more than 9 nucleoside units it is lower than in the case of dinucleotides. Apparently, a methylphosphonate DNA strand cannot easily assume the right-handed conformation of the sugar phosphate backbone required for forming a stable duplex.

Our group has synthesized the hexanucleotide d([Tp(OMe)]₅T), in which methylphosphate groups having the formula -O-P(O)(OMe)-O- act as linking elements between the nucleoside units. As described by Koole et al, Proceedings of the Koninklijke Nederlandse Akademie van Wetenschappen, series B, 89 (1986) 51-55, this substance is capable of forming a stable parallel duplex with itself, i.e. with hydrogen bonds between thymine bases. The Tₘ value of the substance consisting of a mixture of diastereomers is at about 67^{o}C. Because the substance only contains thymine bases which do not carry a primary amino group, the substance could be prepared by methylation of the phosphate groups with methyl methanesulphonate as described by Rhaese and Freese, Biochim. Biophys. Acta 190 (1969) 418-433.

Conventional acyl blocking groups of the amino moieties of the bases A, C and G have to be removed by treatment with concentrated ammonia, which treatment will also result in destruction of the base-labile phosphate triester groups. This poses a problem to the synthesis of poly(deoxyribonucleotides) with methylated phosphate groups, which comprise A, C and/or G bases.

The problem can be avoided by using the 9-fluorenylmethyloxycarbonyl (Fmoc) group for masking the amino groups of the bases. Said Fmoc group is described by Heikkilä and Chattopadhyaya in Acta Chem. Scand. B37 (1983) 263-265 and can be cleaved off via a mild β-elimination. Said β-elimination is based on the acidic nature of the proton on the β-carbon atom and is effected by treatment with e.g., triethylamine, which does not affect the methylated phosphate groups in the DNA backbone. Thus, it became possible to prepare any desired poly(deoxyribonucleotide) with methylated phosphate groups.

In the meantime McBride et al, J.A.C.S. 108 (1986) 2040-2048 have proposed the use of base protecting amidine groups in the synthesis of oligonucleotides which can be easily and selectively removed by treatments with 1,2-ethanediamine/phenol/water without simultaneously deprotecting methylated phosphate groups. Making use thereof, we have developed a synthesis method which renders it possible to prepare any desired poly(deoxyribonucleotide) with methylated phosphate groups. By means of this synthesis method we have prepared a number of poly(deoxyribonucleotides) with methylated phosphate groups, and during hybridisation experiments we have surprisingly found that with complementary DNA molecules the compounds form antiparallel duplexes, the stability of which continues to increase with increasing chain lengths. In view of the instability of duplexes having greater chain lengths, as found in methylphosphonate polynucleotides, it is surprising indeed that replacement of the methyl group bound to phosphorus by a methoxy group has such a drastic effect on the stability of the duplex. In the case of methylphosphate polynucleotides, duplexes having lengths of 2, 3, 6 and 8 nucleoside units per chain show Tₘ values of respectively about 30^{o}C, 41^{o}C, 67^{o}C and 85^{o}C.

This surprisingly high stability, particularly in the case of longer molecules, can be utilized in many ways, e.g., in hybridisation steps in which there is a need for forming stable duplex molecules by using a methylphosphate polynucleotide as a probe. Such hybridisation steps not only form part of several scientific and practical DNA recombinant procedures, but can also be used in diagnostic applications to establish the presence of a certain DNA sequence in samples to be analyzed. Because also longer duplex molecules are stable, molecules can be used having a base sequence specific for a certain type of DNA, e.g., for the DNA of a certain bacterium, a certain virus, a certain eucaryotic parasite, a certain type of tumor cell, or in a more general sense, of cells having a changed DNA, as occurring with dementia, muscular dystrophy, autoimmune diseases and other diseased conditions. Not only for diagnostic purposes can the methylphosphate poly(deoxyribonucleotides) according to the invention be used, but also for therapeutic purposes. Because with the DNA present in the cell they form stable duplexes having a Tₘ value above the body temperature in the case of chain lengths of 3 or more nucleoside units, they are capable of disturbing replication (and therefore cell multiplication) and transcription (and therefore a normal activity of the cell), while in the case of substances having a base sequence specific for certain DNA a directed treatment of bacterial or viral infections, eucaryotic parasites, cancer cells and other diseased conditions associated with changed DNA, such as dementia, muscular dystrophy and autoimmune diseases can be realized. It turns out that the methylphosphate poly(deoxyribonucleotides) according to this invention are not decomposed in vivo, and because of their non-anionic character they can easily pass through cell membranes, so that they can reach in active form the DNA present in the cell. The compounds according to this invention could also be used for genotherapeutic purposes. As regards the organisms to be treated, the invention is not limited to human beings but is equally applicable to animals and plants.

It could further be considered to make use of the property that the compounds show a temperature-dependent UV absorption which can be adjusted to the desired temperature behaviour through a selection of the length of the molecule.

The invention first resides in the novel compounds themselves, i.e. in poly(deoxyribonucleotides) comprising 8 or more deoxyribonucleoside units which are linked together by phosphate groups, characterized in that at least part of the phosphate groups is methylated, with the proviso that poly (deoxyribonucleotides) which are covalently bound to a solid support are excluded.

Longer molecules are preferred because of a higher stability of the duplex and the possibility of being specific for certain DNA. Molecules having 8 or more nucleoside units show a Tₘ value which is clearly above the body temperature of mammals, including man. At chain lengths of 8 or more, preferably 10 or more, e.g. 15 to 18 nucleoside units, a high specificity is obtainable for certain DNA.

In order to obtain a high stability, it is not always necessary that all phosphate groups should be methylated, and for economical reasons it may be desirable that only a part of the substance contains methylphosphate groups. However, compounds that do not contain charged phosphate groups are preferred because these show the highest duplex stability and most easily pass through cell membranes.

Preferred are compounds having formula 1 of the sheet of formulae, in which n is an integer of 7 or more, R¹ and R² are independently of each other hydrogen, -CH₃, -C₂H₅, -COCH₃ or -PO(OCH₃)₂, and each X is, independently of each other, a base from the group consisting of adenine, guanine, thymine and cytosine.

The poly(deoxyribonucleotides) according to the invention preferably have a base sequence specific for the DNA of a bacterium or virus or eucaryotic parasite or for the DNA of a tumor cell.

The invention further resides in pharmaceutical compositions which, in addition to at least one carrier suitable for therapeutic application, comprise at least one poly(deoxyribonucleotide) according to the invention. The form of these pharmaceutical compositions depends on the selected route of administration. The selection of the carrier and other conventional components of the composition are deemed to be within reach of those skilled in the art. The compounds according to the invention will preferably be used in the form of solutions, emulsions or suspensions for, e.g., intravenous, intramuscular or intralesional administration.

The invention also resides in the use of the novel compounds according to the invention for diagnostic purposes and, in a broader sense, for hybridisation purposes.

The invention likewise resides in a process for preparing the novel compounds according to the invention, comprising
a. preparation of a deoxyribonucleoside having formula 2 of the sheet of formulae, in which Y is a 3' OH protecting group, and X* is thymine, or adenine, guanine or cytosine with a base protecting amidine or Fmoc group,
b. preparation of one or more compounds having formula 3 of the sheet of formulae, in which NR₂ is an activatable leaving-group, D is a 5' OH protecting group, and X* is thymine, or adenine, guanine or cytosine with a base protecting amidine or Fmoc group,
c. activation of a compound having formula 3 and coupling to the free 5' OH group of the compound having formula 2,
d. oxidation of the phosphite group in the resulting reaction product into a phosphate group,
e. removal of the 5' OH protecting group D,
f. chain elongation, if required, by repeatedly adding a novel deoxyribonucleoside methylphosphate subunit, according to steps c, d and e,
g. conversion, if desired, of the 5' OH group into an -OR¹ group,
h. conversion, if desired, of the protected 3' OH group into an -OR² group, and
i. removal of the base protecting amidine or Fmoc groups, if present.

In step a of this process any suitable 3' OH protecting group can be used. Preferably, however, Y is an acetyl group. In step b any suitable 5' OH protecting group can be used, but a di-p-methoxytrityl group is preferred. The leaving group NR₂ is a group having a great stability but likewise capable of being easily activated. Preferred is a diisopropylamino group which can be easily activated with heterocyclic compounds, such as preferably tetrazole.

In this process an amidine group may be used for protecting the bases adenine, guanine and cytosine. This amidine group preferably has the formula -N=C(R³)-NR⁴R⁵, in which R³ and R⁴ are independently of each other hydrogen or alkyl having 1-6 carbon atoms, and R⁵ is alkyl having 1-6 carbon atoms. However, R⁴ and R⁵ or R³ and R⁵, together with the nitrogen atom or the nitrogen and carbon atoms to which they are bonded, may also form a heterocyclic ring having 4-7 atoms in the ring.

Preferably, the base protecting amidine group has the formula -N=C(CH₃)-N(CH₃)₂, which group may be introduced by a conversion with the compound 1-dimethylamino-1,1-dimethoxy-ethane.

The removal of this amidine group in step i preferably occurs with ethylenediamine.

If the bases adenine, guadine and/or cytosine were protected by the Fmoc group, removal may be effected preferably by treatment with triethylamine.

However, also other methods of preparation can be used, e.g., an automated phosphite triester method as described before, which requires, however, a modification so as to avoid that when the synthesized molecule is released from the solid carrier chain rupture occurs owing to the methylphosphate groups present. The conventional treatment with ammonia is not suitable for this reason. This problem can be overcome by using another reagent which releases the molecule from the carrier without attacking the integrity of the synthesized molecule or by using another spacer which is selectively removable at the desired moment without attacking the integrity of the synthesized molecule.

Similar considerations apply to other methods, such as the well-known phosphate triester method: appropriate modifications will render them useful for the preparation of the poly(deoxyribonucleotides) according to the invention.

The invention will now be illustrated in more detail by means of an experimental part.

### Preparation of: d(acAp(OMe)Ap(OMe)Aac) having formula 4 and d(acAp(OMe)Ap(OMe)Ap(OMe)Aac) having formula 10 (see scheme A)

Starting from the phosphoramidite having formula 5, prepared according to McBride et al. (J. Am. Chem. Soc. 105 (1986) 2040), and the modified nucleoside having formula 6, the dinucleotide having formula 7 is prepared in a three-step synthesis. The three steps are: 1. coupling; 2. oxidation of phosphite into phosphate; 3. deprotection of the 5' terminal (detritylation). The three steps are carried out as follows:

### (1). Coupling

The compounds (5) and (6) are combined in equimolar amount. Dry toluene is added twice to remove all the water azeotropically. The reagents are then dissolved in dry pyridine. Addition of 5 equivalents of tetrazole permits the reaction to start. After 1 hour the conversion has been completed.

### (2) Oxidation

The phosphite is oxidized by adding I₂ (1.5 equivalent) dissolved in acetonitrile/lutidine/water (3:2:1 volume ratio). After 10 minutes ethyl acetate is added to the reaction mixture. Then extraction is effected with a 1% solution of NaHSO₃ in water.

The organic phase is dried, filtered, and concentrated until a viscous oil is formed. Purification of the product takes place by means of column chromatography with dichloromethane/methanol (95:5) as eluent. Yield: 84%.
R_{f} (dichloromethane/methanol 90:10) : 0.40.
Rf (dichloromethane/methanol 95:5) : 0.05.

### (3) Detritylation

The product of the oxidation reaction is dissolved in acetic acid/water (4:1), with subsequent stirring for 2 hours at 50^{o}C. Then 5% ammonium acetate in water is added whereby dimethoxytrityl alcohol precipitates. The precipitate is removed by filtration. Purification of the product is effected by column chromatography. Yield of (7) : 81%.
R_{f} (dichloromethane/methanol 90:10) : 0.26.

Starting from (7) the three-step synthesis may be repeated whereby the trinucleotide (8) is obtained. A next repetition gives the tetranucleotide (9).

During the synthesis of the trinucleotide there was found:
R_{f} of the product after oxidation (dichloromethane/methanol 90:10) : 0.24.
R_{f} after detritylation (8) (dichloromethane/methanol 90:10) : 0.14.

During the synthesis of the tetranucleotide there was found:
R_{f} of the product after oxidation (dichloromethane/methanol 95:5) : 0.09.
R_{f} after detritylation (9) (dichloromethane/methanol 90:10) : 0.11.

Both (8) and (9) are acetylated at the 5' terminal by reaction with a two-fold excess of acetic anhydride in dry pyridine. The amidine groups on the adenine bases are removed according to the prescription of McBride et al. (J. Am. Chem. Soc. 108, 2040) by reaction with 1,2-ethanediamine/phenol/water.

The products are obtained in pure form by means of two-dimensional thin layer chromatography.

For the purposes of comparison an unmodified trinucleotide d(ApApA) was prepared via the standard phosphoramidite route on an automated DNA synthesizer.

### Comparison of the compounds

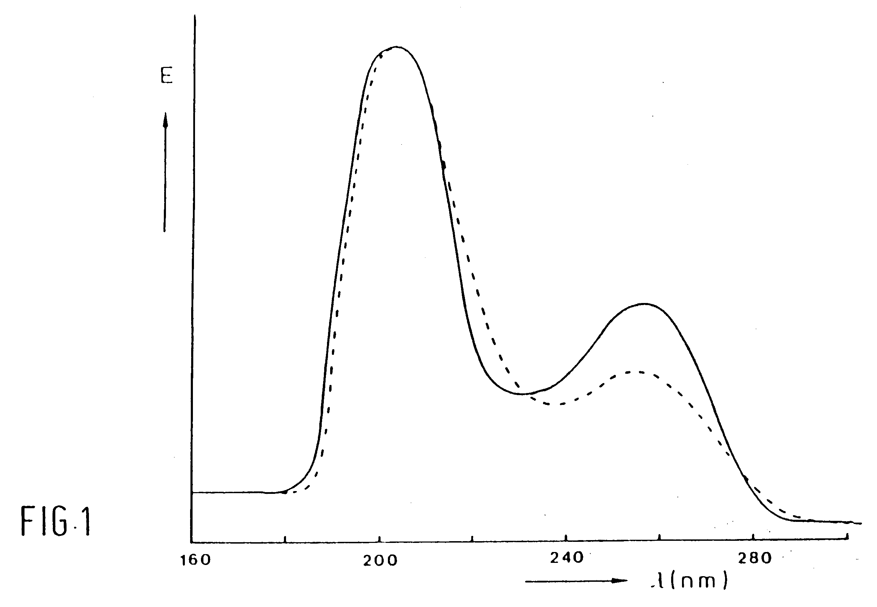

Neutral solutions in water of d(acAp(OMe)Ap(OMe)Aac) and d(ApApA) showed nearly identical UV absorption spectra (fig. 1). In both cases the typical absorption maximum was at 257 nm. For the compound according to the invention the minimum was at 230 nm; A₂₅₀/A₂₆₀ was 0.94; and A₂₈₀/A₂₆₀ was 0.12. For the normal trinucleotide the minimum was at 236 nm; A₂₅₀/A₂₆₀ was 1.08; and A₂₈₀/A₂₆₀ was 0.22.

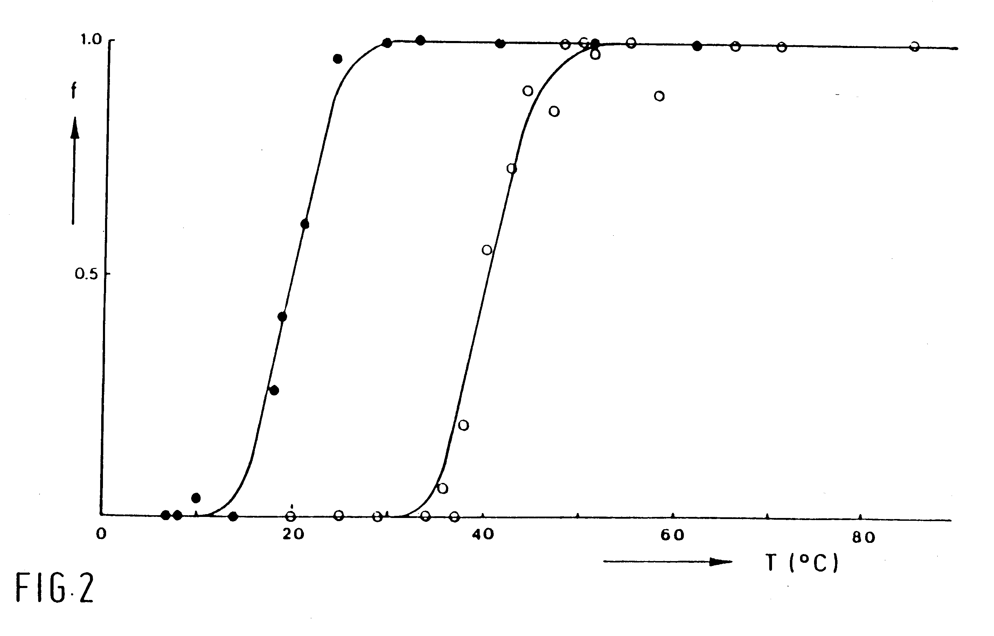

Hybridisation tests with poly(dT) comprised an analysis of the UV hyperchromicity as a function of the temperature of the sample. With poly(dT) the compound according to the invention formed a duplex which dissociated at a melting temperature Tₘ of 41^{o}C. The duplex of d(ApApA) with poly(dT) dissociated at a Tₘ of 20^{o}C under the same testing conditions (fig.2).

It appears from fig. 2 that the melting transitions are equally sharp in both cases, i.e. the helix ⇄ coil transition was completed in both cases within a temperature range of about 20^{o}C. Accordingly, all of the four different diastereomers of the compound according to the invention have the same Tₘ value of 41^{o}C, from which it may be concluded that the configuration of the methylphosphate groups (Rₚ and Sₚ) has no effect on the stability of the duplex.

It further appears from fig. 2 that methylation of the phosphate groups gives a shift of the melting curve of +21^{o}C.

Similar experiments showed that the Tₘ value of compounds according to the invention increases with increasing length of the molecules. The Tₘ value of a duplex with a dinucleotide according to the invention was at about 30^{o}C, while duplexes with a hexanucleotide according to the invention had a Tₘ value of about 67^{o}C and duplexes with an octanucleotide according to the invention had a Tₘ value of about 85^{o}C.

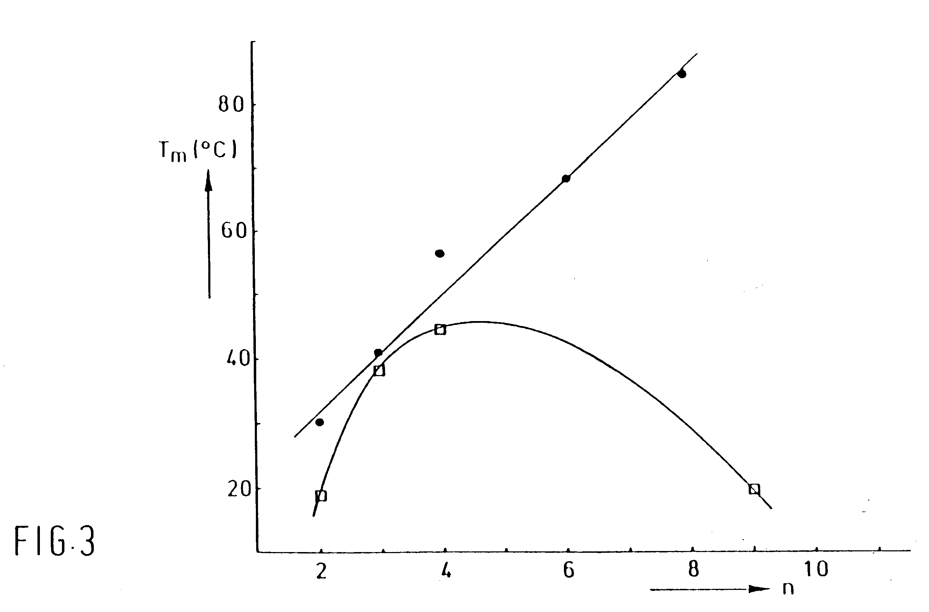

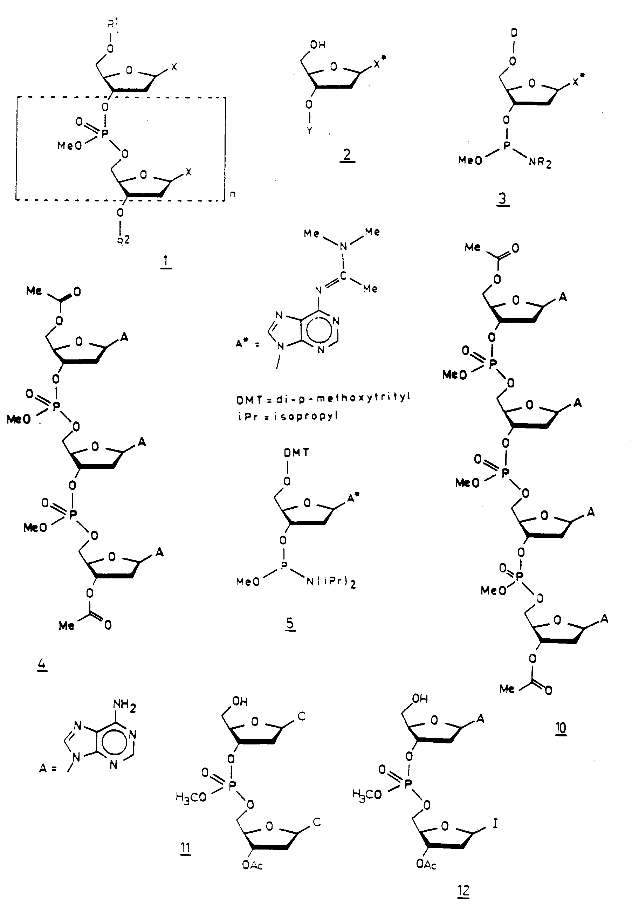

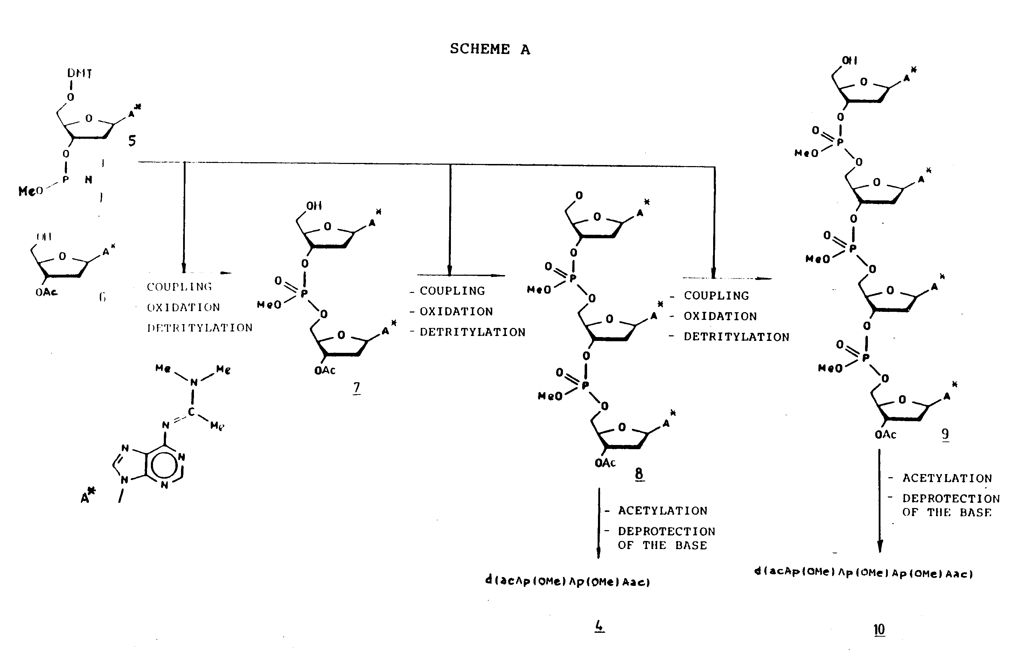

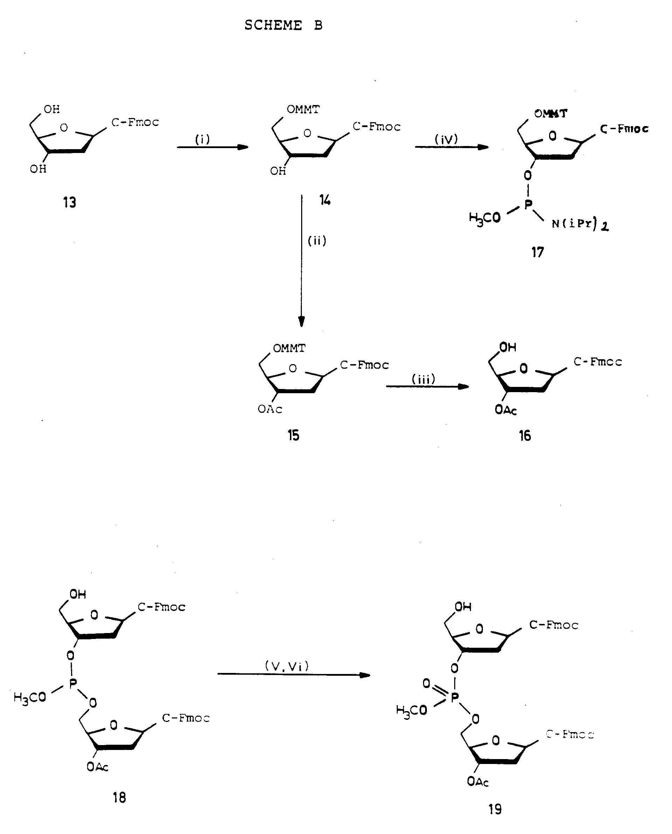

It appears from fig. 3 that the Tₘ value of the compounds according to the invention increases with increasing chain length, while the phosphonates (curves with open squares) having greater chain lengths form less and less stable duplexes.

Extended UV hyperchromicity experiments on the hybridization of d(acAp(OMe)Ap(OMe)Aac) have revealed that duplex formation occurs exclusively with poly(dT) and not with poly(U) (the complementary RNA). This specificity is essentially based on the fact that methyl phosphate DNA intrinsically matches the righthanded B DNA geometry. A biological experiment was performed to determine the impact of d(acAp(OMe)Ap(OMe)Aac) on DNA synthesis and protein synthesis in rat fibroblast cells. It was found that DNA synthesis is markedly retarded (80% inhibition at a concentration of d(acAp(OMe)Ap(OMe)Aac) of 10 µM), while protein synthesis remained essentially unaffected. This is explained by the affinity of d(acAp(OMe)Ap(OMe)Aac) for natural DNA, and not for natural RNA.

### Use of the Fmoc group

The utility of the Fmoc group was tested in the synthesis of the phosphate-methylated dinucleotides 11 and 12. 5'-(4-Methoxytriphenylmethyl)-dC-Fmoc (14) was the key precursor for 11 (Scheme B). Compound 14 was prepared from dC-Fmoc (13) and 4-methoxytriphenylmethyl chloride in dry pyridine. Subsequently, 14 was treated with acetic anhydride to form 15, from which the 3'-terminal building block 16 was obtained through detritylation in 80% acetic acid. Another portion of 14 was reacted with 1.1 equivalent of methoxy bis (diisopropylamino) phosphine (δ ³¹P in CDCl₃, 131.6 ppm), in the presence of 1H-tetrazole in a strictly water-free pyridine medium, yielding the coupling synthon 17 in situ. This was evident from the ³¹P NMR spectrum of a sample of the reaction mixture, showing two peaks at δ 150.0 and 149.4 ppm in a 1 : 1 ratio, due to two diastereomeric forms of 17. No residual ³¹P NMR resonance at δ 131.6 ppm was found. The formation of 17 is essentially based on the fact that alkoxy bis (dialkylamino) phosphines are selectively activated by weak acids such as 1H-tetrazole. The solution of 17 was immediately reacted further with a solution of 16 (1.05 equivalent with respect to 17), and tetrazole in dry pyridine. This readily furnished the diastereomeric phosphite triester 18, as judged by ³¹P NMR (δ 140.8 and 140.5 ppm in CDCl₃), and thin layer chromatography. The corresponding methyl phosphate triesters (δ 0.4 and 0.3 ppm in CDCl₃), were formed quantitatively upon reaction with tert-butyl hydroperoxide. Subsequent detritylation with 80% acetic acid afforded the partially protected dinucleotide 19, which was isolated as a white powder after column chromatography using a mixture of chloroform and methanol (9 : 1) as eluent (R_{f} = 0.65). The two diastereomeric forms of 19 showed ³¹P NMR resonances at δ 0.1 and -0.4 ppm in CDCl₃. Compound 19 was stirred for 14 h in a 1 : 1 mixture of chloroform and triethylamine, which led to deblocking of the C-bases. The target compound 11 precipitated during this reaction, (δ ³¹p in CD₃OD, 4.0 and 3.4 ppm).

The synthesis of the phosphate-methylated dinucleotide 12 was performed in essentially the same way, using 5'-(4-methoxytriphenylmethyl)-dA-Fmoc and 3'-O-acetylthymidine as building blocks. The former compound was first reacted with 1.2 equivalent of methoxy bis (diisopropylamino) phosphine in a water-free medium of dichloromethane and acetonitrile in the presence of 1H-tetrazole. This readily afforded the corresponding in situ coupling synthon (δ ³¹P in CDCl₃, 149.7 and 149.5 ppm). A solution of 3'-O-acetylthymidine (1.2 equivalent with respect to 5'-(4-methoxytriphenylmethyl)-dA-Fmoc), and 1H-tetrazole was then added to the reaction mixture, to furnish the dinucleoside phosphite dA-5'-P-3'-dT (δ ³¹p in CDCl₃, 141.3 and 140.4 ppm). After oxydation with tert-butyl hydroperoxide, the corresponding methyl phosphate triester (δ ³¹P in CDCl₃, -0.05 and -0.53 ppm) was obtained. This product was isolated as a white solid after column chromatography, using 2-butanone as eluent (R_{f} = 0.28). The 4'-methoxytriphenylmethyl group was cleaved in 80% acetic acid. Finally, the Fmoc group was removed by treatment with a mixture of triethylamine (10 equivalents with respect to the substrate) and pyridine, to yield compound 12, with ³¹P NMR signals at -0.4 and -0.7 ppm in CDCl₃.

The scope of the Fmoc approach to phosphate-methylated DNA oligomers can evidently be extended to systems of an arbitrary nucleotide sequence.

Following the Fmoc approach, the following methyl phosphate DNA fragments have also been synthesized: d(Cp(OMe)Cp(OMe)Cac), d(Tp(OMe)Cp(OMe)Cac) and d(Tp(OMe)Tp(OMe)pAp(OMe)pAac). UV hyperchromicity experiments on the hybridization of d(Cp(OMe)Cp(OMe)Cac) with natural poly(dG) have shown a Tm value of 55^{o}C (compare: Tm = 41^{o}C for d(acAp(OMe)Ap(OMe)Aac) and poly(dT)). It can be concluded that hybridization via C - G base pairs is substantially stronger than hybridization via A - T base pairs. Thus, the stability of the complex is a function of both the length and the base sequence.

### Biological activity

The alleged biological activity of methyl phosphate DNA was confirmed by two in vitro cell growth experiments: (i) 100 µM d(acAp(OMe)Ap(OMe)Aac) with E. coli cells (K12AB 3292 and K12JM 101A) resulted in 100% inhibition of colony formation. The low inhibitory concentration shows that the influence goes beyond a general toxic effect: (ii) a small but significant inhibition (15 - 20%) of cell growth of human ovarium malign cells has been induced by methyl phosphate d(acAp(OMe)Ap(OMe)Aac) in a concentration of 150 µM. Elongation of the methyl phosphate DNA will surely enhance activity and selectivity with respect to cell growth inhibition.

## Claims

1. Poly(deoxyribonucleotides) comprising 8 or more deoxyribonucleoside units which are linked together by phosphate groups, characterized in that at least part of the phosphate groups is methylated, with the proviso that poly(deoxyribonucleotides) which are covalently bound to a solid support are excluded.

2. Poly(deoxyribonucleotides) according to claim 1 having the formula 1: 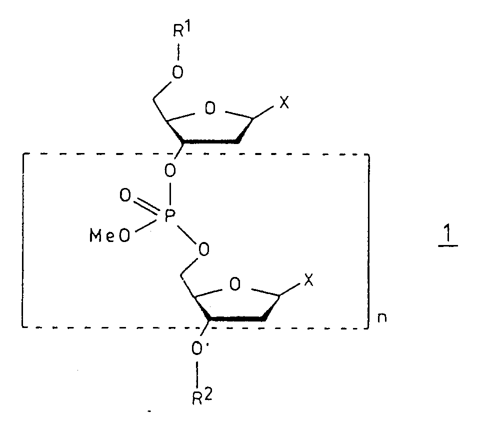 wherein n is an integer of 7 or more, R¹ and R² independently represent hydrogen, -CH₃, -C₂H₅, -COCH₃ or -PO(OCH₃)₂, and each X independently represents a base from the group consisting of adenine, guanine, thymine and cytosine.

3. Poly(deoxyribonucleotides) according to claim 1 or 2, having a base sequence which is specific for the DNA of a bacterium or virus or eukaryotic parasite or tumor cell.

4. Pharmaceutical compositions comprising at least one carrier suitable for therapeutic application and at least one poly(deoxyribonucleotide) according to any of claims 1-3.

5. Use of poly(deoxyribonucleotides) according to any of claims 1-3 for diagnostic purposes.

6. Use of poly(deoxyribonucleotides) according to any of claims 1-3 for hybridisation purposes.

7. Process for preparing poly(deoxyribonucleotides) according to any of claims 1-3, comprising
(a) preparation of a deoxyribonucleoside having formula 2: 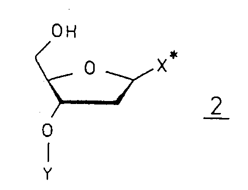 wherein Y is a 3'-OH protecting group and X* is thymine, or adenine, guanine or cytosine with a base protecting amidine or Fmoc group,
(b) preparation of one or more compounds having formula 3: 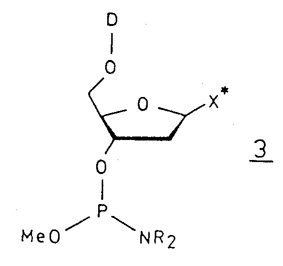 wherein NR₂ represents an activatable leaving group, D is a 5'-OH protecting group and X* is thymine, or adenine, guanine or cytosine with a base protecting amidine or Fmoc group,
(c) activation of a compound having formula 3 and coupling to the free 5'-OH group of the compound having formula 2,
(d) oxidation of the phosphite group in the resulting reaction product into a phosphate group,
(e) removal of the 5'-OH protecting group D,
(f) chain elongation by repeatedly adding further deoxyribonucleoside methylphosphate subunits according to steps (c) to (e),
(g) conversion, if desired, of the 5'-OH group into an -OR¹ group,
(h) conversion, if desired, of the protected 3'-OH group into an -OR² group, and
(i) removal of the base protecting amidine or Fmoc groups, if present.

8. Process according to claim 7, comprising in step (a) the use of an acetyl group as 3'-OH protecting group Y and in step (b) the use of a di-p-methoxytrityl group as 5'-OH protecting group D and the use of a diisopropylamino group as leaving group NR₂.

9. Process according to claim 7 or 8, comprising in step (c) an activation of the compound having formula 3 with tetrazole.

10. Process according to any of claims 7-9, which comprises protecting the bases adenine, guanine and cytosine by converting the primary amino group into a group having the formula
-N=C(R³)-NR⁴R⁵
wherein R³ and R⁴ independently are hydrogen or C₁-C₆ alkyl, R⁵ is C₁-C₆ alkyl, or either R⁴ and R⁵, or R³ and R⁵, together with the nitrogen atom to which they are attached, or together with the nitrogen and carbon atoms to which they are attached, form a heterocyclic ring having 4-7 atoms in the ring.

11. Process according to claim 10, which comprises protecting the bases adenine, guanine and cytosine by a reaction with the compound 1-dimethylamino-1,1-dimethoxyethane to convert the primary amino group of the bases into a group having the formula
-N=C(CH₃)-N(CH₃)₂

12. Process according to any of claims 7-11, which comprises removing the base protecting amidine groups in step (i) by treatment with ethylenediamine.

## Patentansprüche

1. Poly(desoxyribonukleotide) mit 8 oder mehr Desoxyribonukleosid-Einheiten, die durch Phosphatgruppen miteinander verbunden sind, dadurch gekennzeichnet, daß wenigstens ein Teil der Phosphatgruppen methyliert ist, unter der Bedingung, daß kovalent an einen festen Träger gebundene Poly(desoxyribonukleotide) ausgeschlossen sind.

2. Poly(desoxyribonukleotide) nach Anspruch 1 der Formel 1: 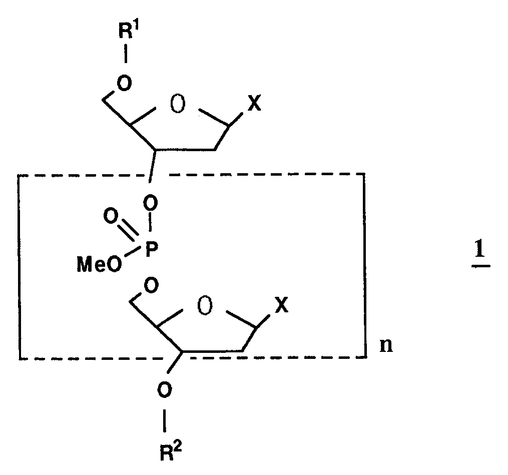 worin n eine ganze Zahl von 7 oder mehr ist, R¹ und R² unabhängig voneinander Wasserstoff, -CH₃, -C₂H₅, -COCH₃ oder -PO(OCH₃)₂ darstellen und jedes X unabhängig voneinander eine Base aus der Gruppe, bestehend aus Adenin, Guanin, Thymin und Cytosin, darstellt.

3. Poly(desoxyribonukleotide) nach Anspruch 1 oder 2, mit einer Basesequenz, die spezifisch für die DNS eines Bakteriums oder Virus oder einer eukaryotischen Parasit- oder Tumorzelle ist.

4. Pharmazeutische Zusammensetzungen, mit wenigstens einem Träger, der für therapeutische Anwendung geeignet ist, und wenigstens einem Poly(desoxyribonukleotid) nach einem der Ansprüche 1 bis 3.

5. Verwendung von Poly(desoxyribonukleotiden) nach einem der Ansprüche 1 bis 3 für diagnostische Zwecke.

6. Verwendung von Poly(desoxyribonukleotiden) nach einem der Ansprüche 1 bis 3 für Hybridisierungszwecke.

7. Verfahren zur Herstellung von Poly(desoxyribonukleotiden) nach einem der Ansprüche 1 bis 3, umfassend:
(a) Herstellung eines Desoxyribonukleosids der Formel 2: 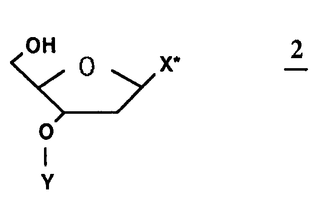 worin Y eine 3'-OH schützende Gruppe ist und X* Thymin, oder Adenin, Guanin, Thymin oder Cytosin mit einem Base schützenden Amidin oder Fmoc-Gruppe ist,
(b) Herstellung einer oder mehrerer Verbindungen der Formel 3: 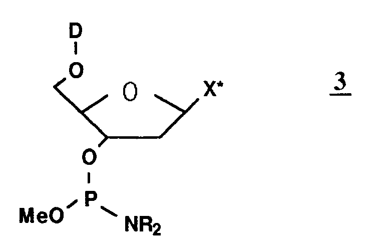 worin NR₂ eine aktivierbare abspaltende Gruppe darstellt, D eine 5'-OH schützende Gruppe ist und X* Thymin, oder Adenin, Guanin oder Cytosin mit einem Base schützenden Amidin oder Fmoc-Gruppe ist,
(c) Aktivierung einer Verbindung der Formel 3 und Kupplung mit der freien 5'-OH-Gruppe der Verbindung der Formel 2,
(d) Oxidierung der Phosphitgruppe im erhaltenen Reaktionsprodukt zu einer Phosphatgruppe,
(e) Entfernung der 5'-OH schützenden Gruppe D,
(f) Kettenverlängerung durch wiederholte Zugabe weiterer Desoxyribonukleosid-Methylphosphat-Untereinheiten nach den Schritten (c) bis (e),
(g) Umsetzung, falls erwünscht, der 5'-OH-Gruppe in eine -OR¹-Gruppe,
(h) Umsetzung, falls erwünscht, der geschützten 3'-OH-Gruppe in eine -OR²-Gruppe, und
(i) Entfernung des Base schützenden Amidins oder der Fmoc-Gruppen, falls vorhanden.

8. Verfahren nach Anspruch 7, umfassend im Schritt (a) die Verwendung einer Acetylgruppe als 3'-OH schützende Gruppe Y und im Schritt (b) die Verwendung einer Di-p-methoxytritylgruppe als 5'-OH schützende Gruppe D und die Verwendung einer Diisopropylaminogruppe als abspaltende Gruppe NR₂.

9. Verfahren nach Anspruch 7 oder 8, umfassend im Schritt (c) eine Aktivierung der Verbindung der Formel 3 mit Tetrazol.

10. Verfahren nach einem der Ansprüche 7 bis 9, umfassend den Schutz der Basen Adenin, Guanin und Cytosin durch Umsetzung der primären Aminogruppe in eine Gruppe der Formel
-N=C(R³)-NR⁴R⁵,
worin R³ und R⁴ unabhängig voneinander Wasserstoff oder C₁-C₆-Alkyl sind, R⁵ C₁-C₆-Alkyl ist, oder entweder R⁴ und R⁵ oder R³ und R⁵, zusammen mit dem Stickstoffatom, an das sie gebunden sind, oder zusammen mit den Stickstoff- und Kohlenstoffatomen, an die sie gebunden sind, einen heterocyclischen Ring mit 4 bis 7 Atomen im Ring bilden.

11. Verfahren nach Anspruch 10, umfassend den Schutz der Basen Adenin, Guanin und Cytosin durch eine Reaktion mit der Verbindung 1-Dimethylamino-1,1-dimethoxyäthan zur Umsetzung der primären Aminogruppe der Basen in eine Gruppe der Formel
-N=C(CH₃)-N(CH₃)₂.

12. Verfahren nach einem der Ansprüche 7 bis 11, umfassend die Entfernung der Base schützenden Amidingruppen im Schritt (i) durch Behandlung mit Äthylendiamin.

## Revendications

1. Polydésoxyribonucléotide comprenant 8 ou davantage d'unités de désoxyribonucléoside liés ensemble par des groupes phosphate, **caractérisés en ce qu'**au moins une partie des groupes phosphate est méthylée, à la condition que les polydésoxyribonucléotides liés par covalence à un support solide sont exclus.

2. Polydésoxyribonucléotide selon la revendication 1 possédant la formule 1: 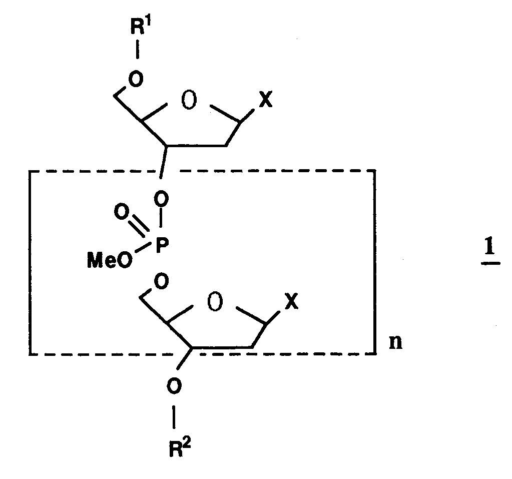 dans laquelle n est un entier valant 7 ou plus, R¹ et R² représentent indépendamment l'hydrogène ou les groupes -CH₃, -C₂H₅, -COCH₃ ou - PO(OCH₃)₂, chaque X représentant indépendamment une base faisant partie du groupe comprenant l'adénine, la guanine, la thymine et la cytosine.

3. Polydésoxyribonucléotide selon la revendication 1 ou 2, possédant une succession de bases spécifique de l'ADN d'une bactérie ou d'un virus ou d'un parasite eucaryote ou d'une cellule tumorale.

4. Formulations pharmaceutiques comprenant au moins un substrat susceptible d'utilisation thérapeutique et au moins un polydésoxyribonucléotide selon l'une des revendications 1-3.

5. Utilisation de polydésoxyribonucléotides selon l'une quelconque des revendications 1-3 dans un but de diagnostic.

6. Utilisation de polydésoxyribonucléotides selon l'une quelconque des revendications 1-3 dans un but d'hybridation.

7. Procédé de fabrication de polydésoxyribonucléotides selon l'une quelconque des revendications 1-3, comprenant les étapes de:
(a) fabriquer un désoxyribonucléoside possédant la formule 2: 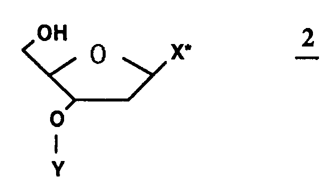 dans laquelle Y est un groupe protecteur 3'-OH et X* est une thymine, une adénine, une guanine ou une cytosine, avec un groupe amidine ou Fmoc protégeant la base,
(b) fabriquer un ou plusieurs composés possédant la formule 3: 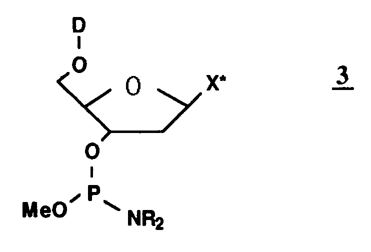 dans laquelle NR₂ représente un groupe labile activable, D est un groupe protecteur 5'-OH et X* est une thymine, une adénine, une guanine ou une cytosine, avec un groupe amidine ou Fmoc protégeant la base,
(c) activer un composé possédant la formule 3 et le lier au groupe 5'-OH libre du composé possédant la formule 2,
(d) oxyder en groupe phosphate le groupe phosphite du produit obtenu au cours de la réaction précédente,
(e) enlever le groupe protecteur D (5'-OH),
(f) allonger la chaîne en ajoutant successivement plusieurs unités de méthylphosphate de désoxyribonucléoside conformément aux étapes (c) à (e),
(g) convertir éventuellement le groupe 5'-OH en un groupe -OR¹,
(h) conversion éventuelle du groupe protecteur 3'-OH en un groupe -OR², et
(i) enlèvement des éventuels groupes amidine ou Fmoc protégeant les bases.

8. Procédé selon la revendication 7, comprenant au cours de l'étape (a) l'utilisation d'un groupe acétyle comme groupe protecteur Y (3'-OH), et en étape (b) l'utilisation d'un groupe di-p-méthoxytrityle comme groupe protecteur D (5'-OH) et l'utilisation d'un groupe di-isopropylamino comme groupe NR₂ labile.

9. Procédé selon la revendication 7 ou 8, comprenant au cours de l'étape (c) l'activation à la tétrazole du composé possédant la formule 3.

10. Procédé selon l'une quelconque des revendications 7-9, comprenant l'opération consistant à protéger les bases adénine, guanine et cytosine en transformant le groupe amino primaire en un groupe possédant la formule:
-N=C(R³)-NR⁴R⁵
dans laquelle R³ et R⁴ représentent indépendamment un hydrogène ou un groupe alkyle de 1 à 6 atomes de carbone, R⁵ étant un groupe alkyle de 1 à 6 atomes de carbone, ou encore R⁴ et R⁵, ou R³ et R⁵, avec l'atome d'azote auquel ils sont liés, ou avec l'atome d'azote et l'atome de carbone auxquels ils sont liés, forment une chaîne hétérocyclique possédant sur la chaîne 4-7 atomes.

11. Procédé selon la revendication 10, comprenant l'opération consistant à protéger les bases adénine, guanine et cytosine par une réaction avec le composé 1-diméthylamino-1,1-diméthoxyéthane en vue de transformer le groupe amino primaire des bases en un groupe possédant la formule:
-N=C(CH₃)-N(CH₃)₃.

12. Procédé selon l'une quelconque des revendications 7-11, comprenant l'opération consistant à enlever au cours de l'étape (i) les groupes amidine protégeant les bases, à l'aide d'éthylènediamine.
